# EUROPEAN PATENT APPLICATION

(11) **EP 2 327 682 A1**
(43) Date of publication of application: **01.06.2011**
(21) Application number: 09013601.1
(22) Date of filing: 29.10.2009
(51) Int. Cl.: C07C 59/11, C07D 309/30, C07D 405/06, C07D 207/337

(54) **Use of amphiphilic compounds for controlled crystallization of statins and statin intermediates**

(71) Applicant: KRKA, D.D., Novo Mesto, 8501 Novo mesto (SI)
(72) Inventor: Kljajic, Alen, 3000 Celje (SI); Zupet, Rok, 1000 Ljubljana (SI)
(74) Representative: Osolnik, Renata

(57) **Abstract**

The invention relates to an improved process comprising amphiphilic compounds for the crystallization of an intermediate used in the process for the preparation of statin intermediates.

## Description

### Field of the Invention

The invention relates to an improved process for the crystallization of organic compounds, which may be intermediates used in the process for the preparation of statin type molecules.

### Background of the Invention and Technical Problem

Statins, of which the representative examples may be selected from rosuvastatin, cerivastatin, atorvastatin, fluvastatin, pitavastatin, bervastatin, dalvastatin or their analogs or pravastatin, simvastatin, lovastatin or their analogs share a characteristic structure, consisting of respectively a heptenoic or heptanoic acid moiety (free acid, salt or lactone) connected to the aromatic or alicyclic core. Biological activity of statins is closely related to their stereochemistry, especially configuration at the chiral atoms of said heptenoic or heptanoic acid moiety.

Crystalline and amorphous statins can be used for the preparation of pharmaceutical formulations. When amorphous active compounds are used it is important to have a technology with an efficient purification step for advanced intermediates or active pharmaceutical ingredient (API), because standard crystallization procedures for purification cannot be applied productively.

Atorvastatin, [R-R*,R*)]-2-(4-fluoro[rho]henyl-[beta],[delta]-dihydroxy-5-(1-methylethyl)-3-phenyl-4-[(phenylamino)carbonyl]-1*H*-pyrrole-1-heptanoic acid, is pharmacologically active as a hypolipidemic and hypocholesterolemic agent. In particular, atorvastatin is useful as a selective and competitive inhibitor of the enzyme 3-hydroxy-3-methylglutaryl- coenzyme A (HMG-CoA) reductase, the rate-limiting enzyme that converts 3- hydroxy-3-methylglutarylcoenzyme A to mevalonate, a precursor of sterols such as cholesterol.

United States Patent Numbers 5,216,174; 5,245,047; 5,248,793; 5,397,792; 5,342,952; 5,298,627; 5,446,054; 5,470,981; 5,489,690; 5,489,691; 5,5109,488; WO97/03960; WO98/04543 and WO99/32434 which are herein incorporated by reference, disclose various processes and key intermediates for preparing atorvastatin.

Fluvastatin, of which the full chemical name is 7-[3-(4-fluorophenyl)-1-(1-methylethyl)-1*H-*indol-2-yl]-3, 5-dihydroxy-hept-6-enoic acid, as well as its sodium salt, are disclosed in EP-A-O 114 027. From the state of the art, methods for the preparation of fluvastatin and its salts are already known, e.g. from K.-M- Cheng e tal. Tetrahedron Letters, 1987, Vol. 28, No. 2, 155-188, or EP 0363 934.

Rosuvastatin or 7-[4-(4-fluorophenyl) -6-(1-methylethyl)- 2-(methyl-methylsulfonyl-amino)-pyrimidin-5-yl]- 3,5-dihydroxy-hept-6-enoic acid, can generally be prepared by any known process such as the processes described in EP 0521471, WO 00/49014, WO 2007/099561 and WO 2004/108691.

Simvastatin ([(1*S*,3*R*,7*R*,8*S*,8*aR*)-8-[2-[(2*R*,4*R*)-4-hydroxy-6-oxo-oxan-2-yl]ethyl)-3,7-dimethyl-1,2,3,7,8,8*a*-hexahydronaphthalen-1-yl]2,2-dimethylbutanoate and lovastatin ([8-[2-(4-hydroxy-6-oxo-oxan-2-yl)ethyl] -3,7-dimethyl-1,2,3,7,8,8a- hexahydronaphthalen- 1-yl] 2-methylbutanoate) are produced by fermentation using microorganisms of different species identified as species belonging to *Aspergillus, Monascus, Nocardia, Amycolatopsis, Mucor* or *Penicillium* genus, and as e.g. described in EP 33538, EP 22478, and DE 3051175.

Chemical purity of the active ingredient is an important factor for the manufacturing of a safe and effective pharmaceutical. The highest possible purity of the product is especially important if the pharmaceutical product should be taken for a longer period as it is the case in the treatment or the preventing of high plasma cholesterol. The accumulation of the impurities from the pharmaceuticals of lower purity could cause side effects during the medical treatment.

It has now been found out that for a number of statins, pertinent structural impurities can easily be removed from the pharmaceutically active ingredient, if the key intermediates are crystallized in the presence of an amphiphilic compound, more preferably a quarternary amine salt, most preferarably cetyltrimethylammonium bromide (CTABr).

The current process for the preparation of the acetonide ester of atorvastatin of formula (Ia) 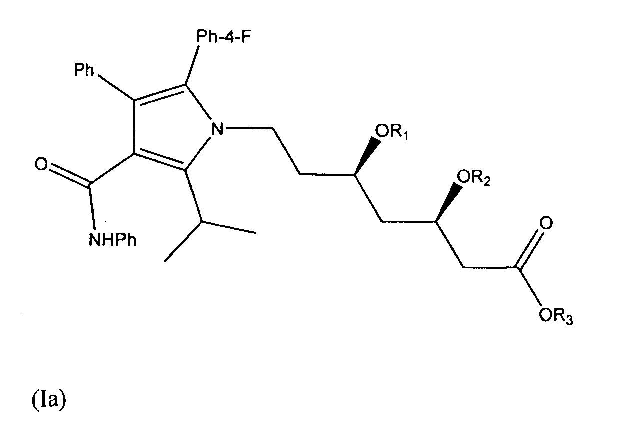 wherein R₁ and R₂ may be the same or different and are selected from any one or more of H, C₁-C₆ alkyl which may be straight or branched, unsubstituted or substituted with a halogen group, C₁-C₆ alkoxy group, or R₁ and R₂ together represent an alkylidene group of the formula CRₐR_{b} wherein Rₐ and R_{b} may be the same or different and are selected from any one or more of a C₁-C₁₁ alkyl group, and R₃ is selected from any one or more of a C₁-C₆ alkyl group, is carried out using a Paal Knorr reaction by reacting the diketone of atorvastatin, 4-fluoro- (2-methyl-1-oxopropyl-[gamma]-oxo-N-[beta]-diphenyl-benzenebutaneamide with the primary amine, alkyl 2-((4R,6R)-6-(2-aminoethyl)-2,2-dimethyl-1,3-dioxan-4-yl)acetate.

In one embodiment of the invention R₁ and R₂ together represent isopropylidene and R₃ is a tertiary butyl.

The product obtained must be purified due to the impurities present, in particular impurity I (hereinafter described as Imp 1): 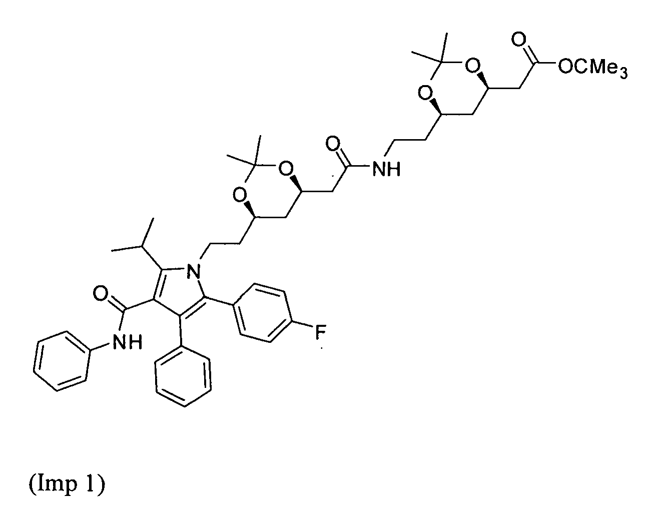 with the chemical name of *tert*-butyl 2-((4*R*,6*R*)-6-{2-[2-((4*R*,6*R*)-6-{2-[2-(4-fluorophenyl)-5-isopropyl-3-phenyl-4-(phenylcarbamoyl)-1*H*-pyrrol-1-yl]ethyl}-2,2-dimethyl-1,3-dioxan-4-yl)acetamido]ethyl}-2,2-dimethyl-1,3-dioxan-4-yl)acetate (C₅₀H₆₄FN₃O₈), and impurity II (hereinafter described as Im2): 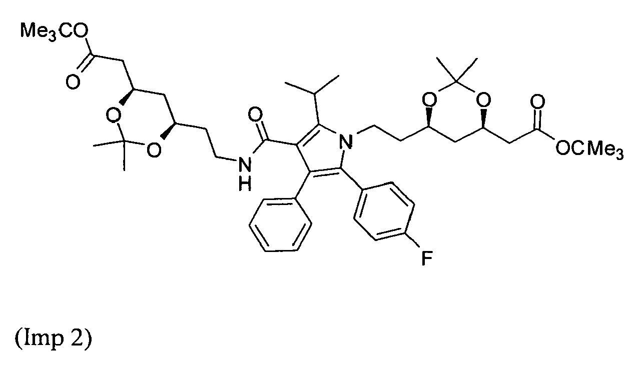 with the chemical name of *tert*-butyl {(4*R*,6*R*)-6-[2-(1-{2-[(4*R*,6*R*)-6-(2-*tert*-butoxy-2-oxoethyl)-2,2-dimethyl-1,3-dioxan-4-yl]ethyl}-5-(4-fluorophenyl)-2-isopropyl-4-phenyl-1*H-*pyrrole-3-carboxamido)ethyl]-2,2-dimethyl-1,3-dioxan-4-yl}acetate (C₄₈H₆₇FN₂O₉).

Similar problems with structural related chemical or sterochemical impurities are encountered also in the crystallization of the following statin intermediates (Ib-Ie): 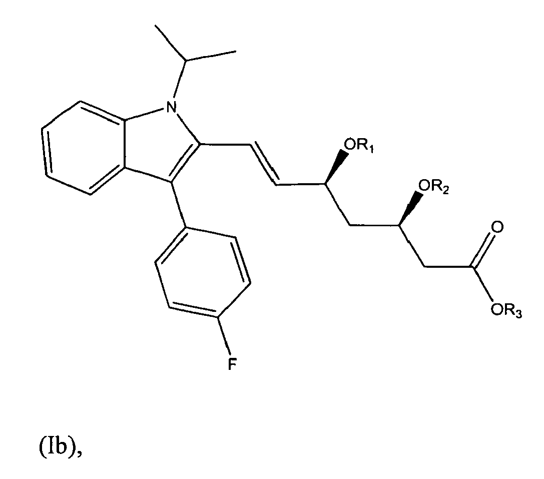 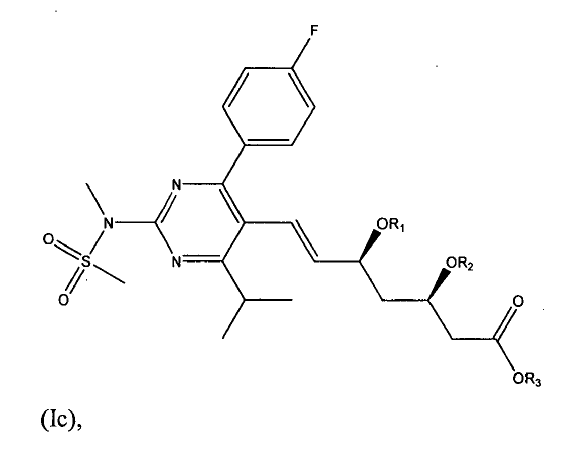 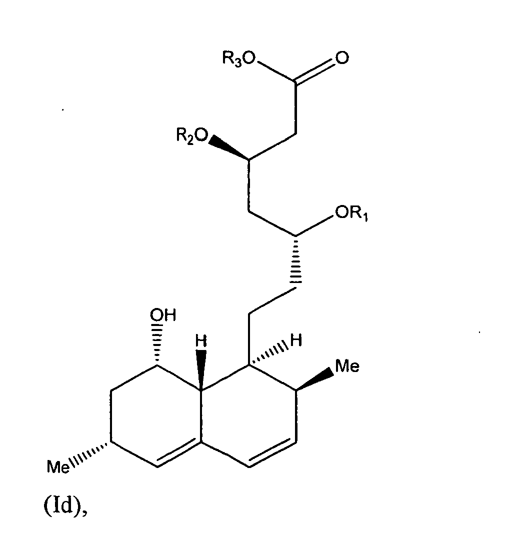 and 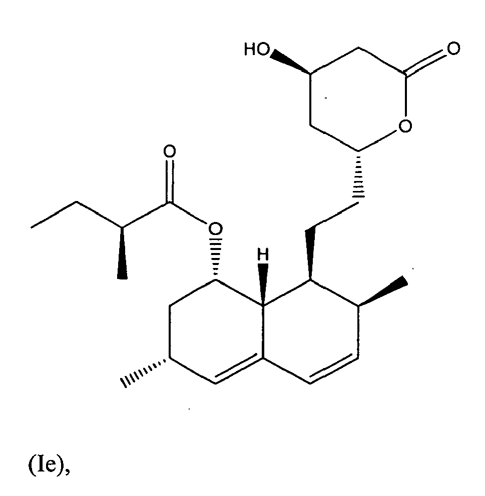 wherein R₁ and R₂ may be the same or different and are selected from any one or more of H, C₁-C₆ alkyl which may be straight or branched, unsubstituted or substituted with a halogen group, C₁-C₆ alkoxy group, or R₁ and R₂ together represent an alkylidene group of the formula CRₐR_{b} wherein Rₐ and R_{b} may be the same or different and are selected from any one or more of a C₁-C₁₁ alkyl group, and R₃ is selected from any one or more of a C₁-C₆ alkyl group; and in case of compound Id may also represent a hydrogen atom.

In one embodiment of the invention R₁ and R₂ together represent isopropylidene and R₃ is a tertiary butyl.

In another embodiment of the invention R₁, R₂ and R₃ in compound Id represent hydrogen atoms H.

Whenever for purification of the statin intermediates the crystallization processes for kinetic polymorphic forms, or amorphous form are preferred over thermodynamic polymorphic form, there is a technical difficulty on industrial scale, because expensive special equipment is needed to cool the crystallization mixture sufficiently quickly in order to prevent transformation to the thermodynamically more stable form. Therefore the technical problem exists to control acceptable filtration properties of the material.

Although it is possible to prepare the kinetic polymorphic form with fast cooling of the crystallization mixture with average cooling rates of reactor jacket temperature at least 1 K/min, adding additional solvent, and subsequently lowering the nucleation temperature, it is not industrially acceptable mainly due to the formation of material with bad filtration properties as a consequence of large jacket-reactor temperature difference (ΔT > 10 °C, smaller particles), low yields of the crystallization processes and larger solvent consumption. The particles produced have relatively small average size diameter and consequently bad filtration properties. Since the processes of isolation are longer due to bad filterability, the transformation from kinetic to thermodynamic crystalline form could happen during the filtration process itself.

There is a need for a robust crystallization process, with technically acceptable cooling rates , e.g. <1 K/min, and leading to a product with good filtration properties (bigger particles) and excellent purity of the isolated product. The optimal and shorter production time and lower solvent consumption subsequently lowers the cost of production.

The problem is solved by the addition of amphiphilic additives, preferably custom-made, especially amines, more preferably quarternary amine salts, e.g. bromides or chlorides, of the present invention.

### Summary of the Invention

According to the invention there is provided a process for the crystallization of a compound of formula Ia-Ie 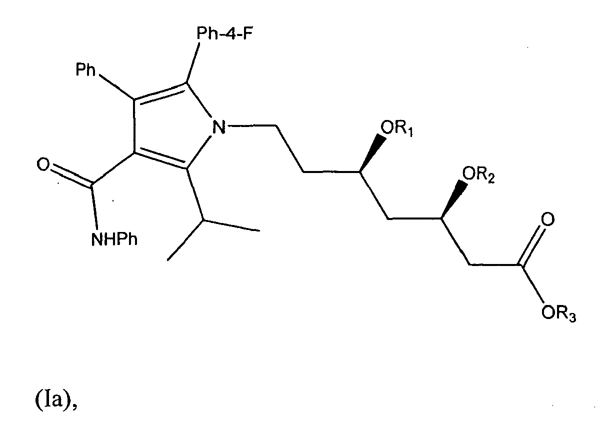 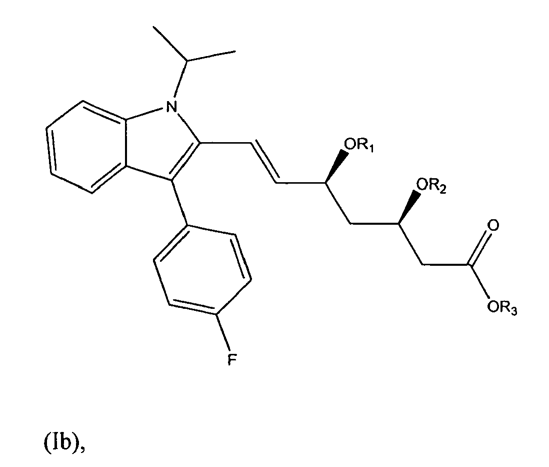 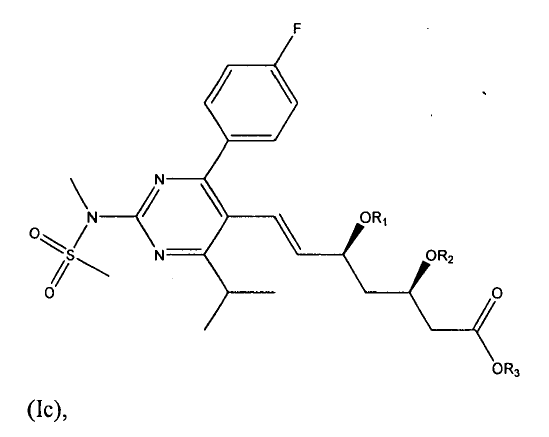 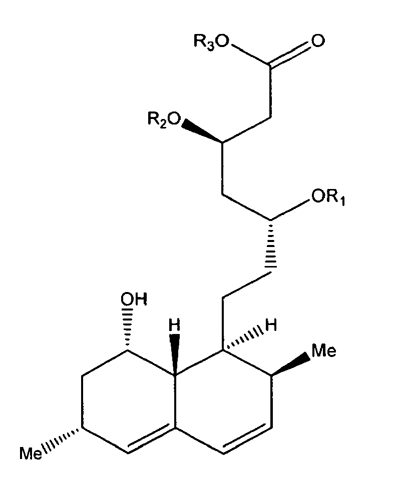 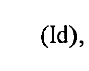 and 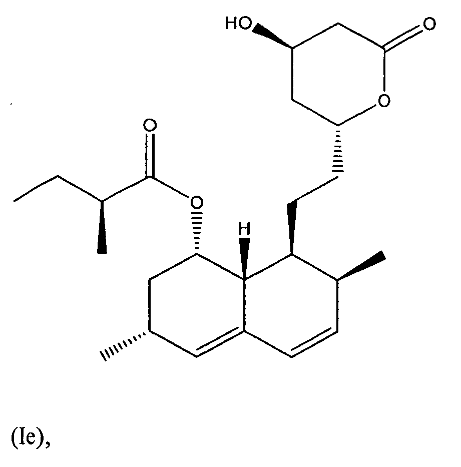 wherein R₁ and R₂ may be the same or different and are selected from any one or more of H, C₁-C₆ alkyl which may be straight or branched, unsubstituted or substituted with a halogen group, C₁-C₆ alkoxy group, or R₁ and R₂ together represent an alkylidene group of the formula CRₐR_{b} wherein Rₐ and R_{b} may be the same or different and are selected from any one or more of a C₁-C₁₁ alkyl group, and R₃ is selected from any one or more of a C₁-C₆ alkyl group; and in case of compound Id may also represent a hydrogen atom, in the presence of an amphiphilic additive, preferably custom-made, especially amine, more preferably quarternary amine salt, most preferarably cetyltrimethylammonium bromide (CTABr).

In one embodiment of the invention R₁ and R₂ together represent isopropylidene and R₃ is a tertiary butyl.

In another embodiment of the invention R₁, R₂ and R₃ in compound Id represent hydrogen atoms H.

The present invention provides an improved crystallization process for preparing the compounds Ia-Ie, preferably the acetonide ester of atorvastatin (*tert*-butyl 2-((4R,6S)-6-((3-(phenylcarbamoyl)-5-(4-fluorophenyl)-2- isopropyl-4-phenyl-1*H*-pyrrol-1-yl)methyl)-2,2-dimethyl-1,3-dioxan-4-yl)acetate), the acetonide ester of rosuvastatin (*tert*-butyl 2-((4R,6S)-6-((E)-2-(4-(4-fluorophenyl)-6-isopropyl-2-(N-methylmethylsulfonamido)pyrimidin-5-yl)vinyl)-2,2-dimethyl-1,3-dioxan-4-yl)acetate), the acetonide ester of fluvastatin (*tert*-butyl 2-((4R,6S)-6-((E)-2-(3-(4-fluorophenyl)-1-isopropyl-1*H*-indol-2-yl)vinyl)-2,2-dimethyl-1,3-dioxan-4-yl)acetate), (3R,5R)-3,5-dihydroxy-7-((1S,2S,6R,8S,8aR)-8-hydroxy-2,6-dimethyl-1,2,6,7,8,8a-hexahydronaphthalen-1-yl)heptanoic acid, and lovastatin Ie.

*Tert*-butyl 2-((4R, 6S)-6-((3-phenylcarbamoyl)-5-(4-flurophenyl)-2-isopropyl-4-phenyl-1*H-*pyrrol-1-yl)methyl)-2,2-dimethyl-1,3-dioxan-4-yl)acetate is preferably prepared by the Paal-Knorr condensation between *tert*-butyl 2-((4R,6R)-6-(2-aminoethyl)-2,2-diemethyl-1,3-dioxan-4-yl)acetate (e.g. prepared by hydrogenation of *tert*-butyl 2-((4R,6R)-6-(cyanomethyl)-2,2-dimethyl-1,3-dioxan-4-yl)acetate in a solvent such as methanol, ethanol or isopropanol in the presence of a catalyst such as Raney Ni and a base such as e.g. aqueous ammonia) and 2-(2-(4-fluorophenyl)-2-oxo-1-phenylethyl)-4-methyl-3-oxo-N-phenylpentanamide in a solvent system such as e.g. heptane/THF (4.1, v/v), catalyzed by pivalic acid; hexane/THF (4.1, v/v), catalyzed by pivalic acid; heptane/THF (4.1, v/v), catalyzed by 2-ethylhexanoic acid; or hexane/THF (4.1, v/v), catalyzed by 2-ethylhexanoic acid. Full details of the reaction are given in US 5280126, US 6476235, US 6545153, and WO 2006/097909.

The *tert*-butyl 2-((4R, 6S)-6-((3-phenylcarbamoyl)-5-(4-flurophenyl)-2-isopropyl-4-phenyl-1H-pyrrol-1-yl)methyl)-2,2-dimethyl-1,3-dioxan-4-yl)acetate obtained may then after purification directly be converted to atorvastatin salt, such as atorvastatin sodium or atorvastatin hemicalcium, or may first be deprotected in e.g. alcohol, acetonitrile, DMF or THF in the presence of an acid such as a.g. acetic acid or aqueous HCl to yield crystalline [R-(R*, R*)]-2-(4-fluorophenyl)-β,δ-dihydroxy-5-(1-methylethyl)-3-phenyl-4-[(phenylamino)-carbonyl]-1H-pyrrole-1-heptanoic acid tertiary butyl ester, which in turn may also be purified by the purification process of the present invention. The latter is then normally converted to the sodium or potassium salt of atorvastatin, e.g. in methanol or ethanol, or their mixtures with water, in the presence of a base such as NaOH or KOH, followed by extractions with an organic solvent such as ether, ethyl acetate or methyl *tert*-butyl ether. The alkaline salt of atorvastatin is converted to the hemi calcium salt by addition of Ca acetate, Ca chloride, Ca(OH)₂ or Ca salt of 2-ethyl hexanoic acid.

The crystallization process with amphiphilic promotors of crystallization according to the present invention enables improved control over particle size of pharmaceutically active compounds and their intermediate leading to improved filterability of the crystallization mixture appropriate for industrial use. Another improvement of the process are lower cooling rates applied in the presence of amphiphilic compounds, preferably tailor-made amphiphilic compounds (especially amines, more preferably quarternary amine salts, most preferably CTABr), smaller differences in jacket-reactor temperature and finally the production of compounds with a higher level of purity. The crystallization process with the amphiphilic additive also has a higher ratio of product/solvent compared to the standard crystallization processes without additive.

The use of amphiphilic additives can modify the ratio between nucleation and crystal growth processes and can result in larger particle size of the produced material compared to materials prepared identically only without the addition of tailor-made group of additives.

Surprisingly, with the use of amphiphilic additives it is possible to operate a crystallization process in an acceptable way for industrial scales using slow cooling ramps, efficient mixing and resulting in desired polymorphic form without inclusion of pertinent impurities in a reliable way. Even more surprisingly, it has been found by the inventors that the use of amphiphilic aditives not only stabilizes the different crystalline forms but also promotes intensive crystal growth process. Large crystalline particles usually have better filtration properties and are desired on a large-scale crystallization process. In particular pronounced was the effect of purification and improved filterability when CTABr as crystallization additive has been used during the crystallization process.

The purity of the obtained compound (Ia-Ie) is higher than 98%, preferably higher than 99%, more preferably higher than 99.9%.

Per crystallization cycle approximately 40-70% (w/w) of the Imp1 present is eliminated is the crystallization is performed with the use of an amphiphilic additive according to the invention, while only 10-25% (w/w) are removed if the crystallization is performed under identical conditions without the use of the amphiphilic additive.

Compounds (Ia-Ie) can further be used for the preparation of any statin form, e.g. atorvastatin free acid, atorvastatin lactone, atorvastatin salt, rosuvastatin free acid, rosuvastatin lactone, rosuvastatin salt, fluvastatin free acid, fluvastatin lactone, fluvastatin salt, simvastatin free acid, simvastatin lactone, or simvastatin salt.

Preferably compounds (Ia-Ie) can further be used for the preparation of any form of a statin salt in its amorphous or any polymorphic form.

Particle size was determined by laser light scattering method using e.g. a Malvern Mastersizer 2000 Apparatus using water as the dilution medium.

The volume average particle diameter was determined by measuring the angular distribution of laser light scattered by a homogeneous suspension of particles.

In the following preferred embodiments of the process are described.

### Brief Description of the Figures

Fig. 1 shows two photographs with amplification 400% and 1000% of *tert*-butyl 2-((4R,6S)-6-((3-(phenylcarbamoyl)-5-(4-fluorophenyl)-2-isopropyl-4-phenyl-1*H*-pyrrol-1-yl)methyl)-2,2-dimethyl-1,3-dioxan-4-yl)acetate crystals obtained according to Examples 1 and 2.
Fig. 2 shows two photographs with amplification 400% and 1000% of *tert*-butyl 2-((4R,6S)-6-((3-(phenylcarbamoyl)-5-(4-fluorophenyl)-2-isopropyl-4-phenyl-1*H*-pyrrol-1-yl)methyl)-2,2-dimethyl-1,3-dioxan-4-yl)acetate crystals obtained according to Comparative example 3.
Fig. 3 shows particle size distribution of *tert*-butyl 2-((4R,6S)-6-((3-(phenylcarbamoyl)-5-(4-fluorophenyl)-2-isopropyl-4-phenyl-1*H*-pyrrol-1-yl)methyl)-2,2-dimethyl-1,3-dioxan-4-yl)acetate crystals obtained according to Examples 1 and 2.
Fig. 4 shows particle size distribution of *tert-*butyl 2-((4R,6S)-6-((3-(phenylcarbamoyl)-5-(4-fluorophenyl)-2-isopropyl-4-phenyl-1*H*-pyrrol-1-yl)methyl)-2,2-dimethyl-1,3-dioxan-4-yl)acetate crystals obtained according to Comparative example 3.

### Detailed Description of the Invention

According to the invention there is provided a process for the crystallization of a compound of formula Ia: 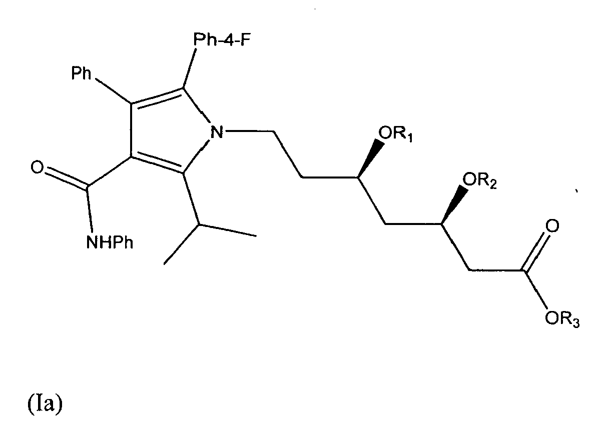 wherein R₁, R₂, and R₃ are as above, in the presence of an amphiphilic additive, preferably amine, more preferably quarternary amine salt, most preferarably cetyltrimethylammonium bromide (CTABr).

In one embodiment of the invention R₁ and R₂ together represent isopropylidene and R₃ is a tertiary butyl.

The present invention provides an improved crystallization process for preparing the acetonide ester of atorvastatin (*tert*-butyl 2-((4R,6S)-6-((3-(phenylcarbamoyl)-5-(4-fluorophenyl)-2-isopropyl-4-phenyl-1*H*-pyrrol-1-yl)methyl)-2,2-dimethyl-1,3-dioxan-4-yl)acetate). The acetonide ester is prepared with improved filterability of the crystallization mixture, high yield and a high level of purity.

Amphiphilic compounds which are used as crystallization additives may custom made and may be chosen from the group of amines, branched or unbranched, unsubstituted or substituted, such as primary amines, secondary amines, tertiary amines or quarternary amine salts; alcohols, preferably C8-C28 alcohols; fatty acids, preferably C8-28 fatty acids; or their amphiphilic derivatives such as e.g. primary amides or salts with an inorganic or organic base. Preferably, the amphiphilic additive is chosen from the group of amines, more preferably from the group of quarternary ammonium salts, alkyl ammonium chloride or bromide, most preferably cetyltrimethylammonium bromide (CTABr).

Solvents may be selected from any one or more of toluene or other aromatic hydrocarbons, tetrahydrofurane, or cyclic and acyclic ethers, ethyl acetate and other esters of alcohols with carboxylic acids, 2- butanone, acetone, and other cyclic and acyclic ketones, methanol, etanol, *iso*-propanol, *n*-propanol, *iso*-butanol, *sec*-butanol, *n*-butanol and other alcohols, acetonitrile, DMSO, DMF and other amides, hexane, heptane, and other acyclic and cyclic aliphatic hydrocarbons. Aliphatic nitriles may also be used as solvents. Binary mixtures of the above may also be used.

The ratio between the compounds Ia-d dissolved and the solvents used is selected between 1g/1mL to 1 g/30 mL, preferably, 1 g/2mL to 1g/10 mL, most preferably between 1 g/3 mL to 1g/6mL.

The weight percentage of the amphiphilic additive used with the regards to the compound I to be crystallized is 0.01 % to 20%, preferably 0.01 % to 10%, most preferably 0.01 % to 1%.

In a first embodiment the amphiphilic additive may be added to the solvent together with compounds Ia-Ie, and heated to a temperature where all of the solute dissolves or the temperature of the reflux of the mixture, and than cooled to a temperature where most of the solute crystallizes.

In a second embodiment, the amphiphilic additive is added to a solution of compounds Ia-Ie already heated to a temperature where all of the solute dissolves or the temperature of the reflux of the mixture, and than cooled to a temperature where most of the solute crystallize.

In a third embodiment of the present invention, the amphiphilic additive is added already to the heated reaction mixture after compounds Ia-Ie had been synthesized and than cooled to a temperature where all of the solute dissolves or the temperature of the reflux of the mixture, and then cooled to a temperature where most of the solute crystallizes.

The average cooling ramp of the reactor jacket (dT/dt) may be chosen from 0.1 K/min to 2 K/min, preferably from 0.1 K/min to 1 K/min, most preferably from 0.1 K/min to 0.5 K/min.

The stirring speed may be chosen from 50 to 400 rpm, preferably from 50 to 300 rpm, most preferably from 100 to 250 rpm.

The crystallized product is isolated by filtration, either by means of a centrifuge or a filter dryer, and dried at a temperature between 50 and 90 °C, preferably between 50 °C and 80 °C, for 5 to 20 hours, preferably for 5 to 15 hours.

The relative filterability of the crystallization mixture in laboratory is determined and compared by means of the laboratory filtration test using Büchner funnel in a suction filtration.

The time needed for the filtration of 10 mL of suspension on a surface of 0.79 cm² of the crystallization mixture according to the present invention is between 8 and 15 seconds, while the time needed for the filtration of a comparable crystallization mixture without the use of an amphiphilic additive is between 70 and 120 seconds.

The chemical purity of the compound Ia obtained was determined with the following method:

### HPLC analysis

Analyses were carried out on Agilent 1100 HPLC system, equipped with gradient pump, variable UV detector, thermostated autosampler and data handling system. Separation was performed on reversed phase C8 column using gradient elution with phosphate buffer pH 3.8 and a mixture of acetonitrile and tetrahydrofurane as organic modifier. Chromatograms were monitored at wavelength 248 nm.

### LC-MS analysis

The analyses were carried out using a Agilent 1200series HPLC system (Agilent, USA) consisting of G1379B degasser, G1312B pump, G1367C autosampler, and a 6330 ion trap mass detector. The chromatographic separation was accomplished on a C₈ column (Ascentis Express 75 mm × 4.6 mm, 2,7µ.m, Supelco) at ambient temperature.

A gradient method was worked out, with acetonitrile content changing linearly during the analysis. The mobile phase A consisted of 5mM ammonium formiate, and mobile phase B consisted of acetonitrile. Gradient:

| time | %B |
|---|---|
| 0 | 10 |
| 16 | 90 |
| 19 | 90 |
| 20 | 10 |

For quantification we used a ion trap mass detector with electrospray ionization in SIM mode (m/z=256).

Compound Ia crystallized by the present invention contained less than 0.10 % of Imp 1 and less than 0.10 % of Imp 2. The amphiphilic additive used for crystallization of compounds I was present in a concentration less than 0.03 % (w/w) in the crystallized compound I.

The chemical purity of the compound Ie obtained was determined with the following method:

### A.) PhEur method for related substances

| HPLC method | |
|---|---|
| **Column:** | Zorbax SB-C8 , 3,5µm particles, 100 x 4.6 mm i.d., |
| **Eluent A:** | 0,1% phosphoric acid |
| **Eluent B:** | acetonitrile |

### Gradient:

| Time (min) | % A | % B |
|---|---|---|
| 0 | 40 | 60 |
| 2 | 40 | 60 |
| 2,8 | 35 | 65 |
| 4,8 | 10 | 90 |
| 6 | 10 | 90 |
| 6,8 | 40 | 60 |
| 9 | 40 | 60 |

| | |
|---|---|
| **Post time:** | 3 min |
| **Flow-rate:** | 1.5 ml/min |
| **Detection:** | UV, 238 nm |
| **Injection volume:** | 5 µl |
| **Column temperature:** | 25°C |
| **Diluent:** | acetonitril |

### Sample preparation:

Accurately weigh about 20 mg of sample into 50 ml volumetric flask, dissolve and dilute to volume with diluent.

### Calculation:

### External standard method

### B.) USP method for dihidro lovastatin

| HPLC method | |
|---|---|
| **Column:** | Zorbax SB-C8 , 5 µm particles, 250 x 4.6 mm i.d., |
| **Eluent A:** | 0,1% phosphoric acid |
| **Eluent B:** | acetonitrile |

**Mobile phase: isocratic:** acetonitril : 0.1 % H₃PO₄ = 65 : 35

| | |
|---|---|
| **Flow-rate:** | 1.5 ml/min |
| **Detection:** | UV, 200 nm |
| **Injection volume:** | 10 µl |
| **Column temperature:** | 40°C |
| **Diluent:** | acetonitril |

### Sample preparation:

Accurately weigh about 25 mg of sample into 25 ml volumetric flask, dissolve and dilute to volume with diluent.

### Calculation:

### External standard method

The present invention is illustrated by the following Examples without being limited thereto.

### EXAMPLES

### I.) Compound Ia

### Example 1

The crystallization is carried out using 2-propanol as medium in a reactor with mechanical stirring. 25g of crude *tert*-butyl 2-((4R,6S)-6-((3-(phenylcarbamoyl)-5-(4-fluorophenyl)-2-isopropyl-4-phenyl-1*H*-pyrrol-1-yl)methyl)-2,2-dimethyl-1,3-dioxan-4-yl)acetate is dissolved in 90 ml of 2-propanol at the reflux temperature of the mixture, 0.12 g of cetyltrimethyl ammonium bromide is added and the temperature is maintained for 30 minutes to allow all solid to dissolve. Then a linear cooling ramp -0.40 K/min of reactor jacket is applied and the mixture is cooled to 3 °C. The formed suspension is homogenized for 2 hours and the product is isolated using Büchner funnel in a suction filtration.

The time needed for the filtration of 10 mL of suspension on a surface of 0.79 cm² was approx. 10s, at a vacuum of about 80-100 mbar.

### Example 2 (large-scale)

The crystallization is carried out using 2-propanol as a medium in a reactor with mechanical stirring. 180 kg of crude *tert*-butyl 2-((4R,6S)-6-((3-(phenylcarbamoyl)-5-(4-fluorophenyl)-2-isopropyl-4-phenyl-1*H*-pyrrol-1-yl)methyl)-2,2-dimethyl-1,3-dioxan-4-yl)acetate is dissolved in 650 L of 2-propanol at the reflux temperature of the mixture, 0.90 kg of cetyltrimethyl ammonium bromide is added and the temperature is maintained for 30 minutes for all solid to dissolve. Then a linear cooling ramp -0.40 K/min of reactor jacket is applied and the mixture is cooled to 3 °C. The formed suspension is homogenized for 2 hours and the product is isolated using a filter-dryer.

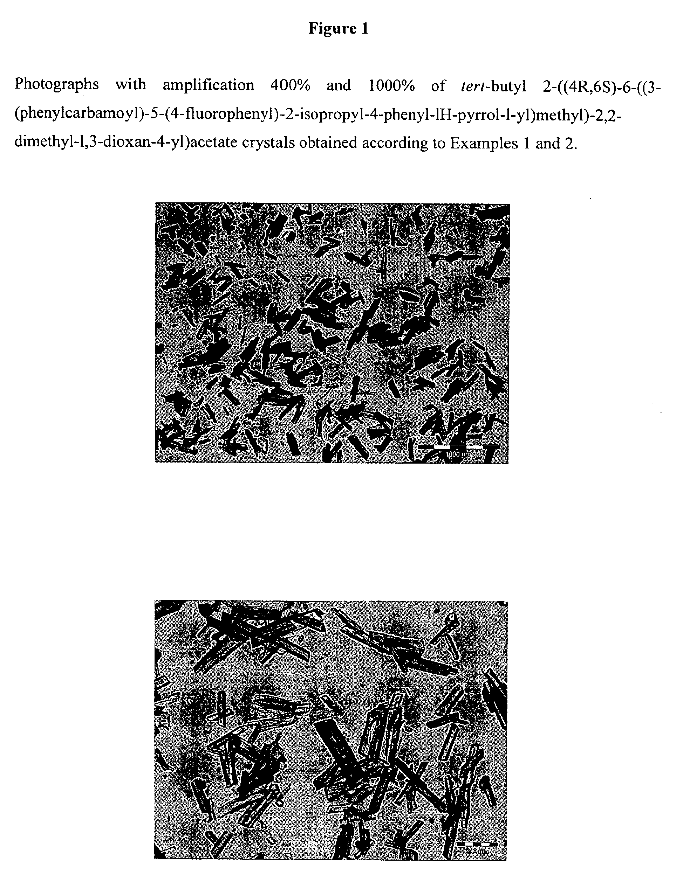

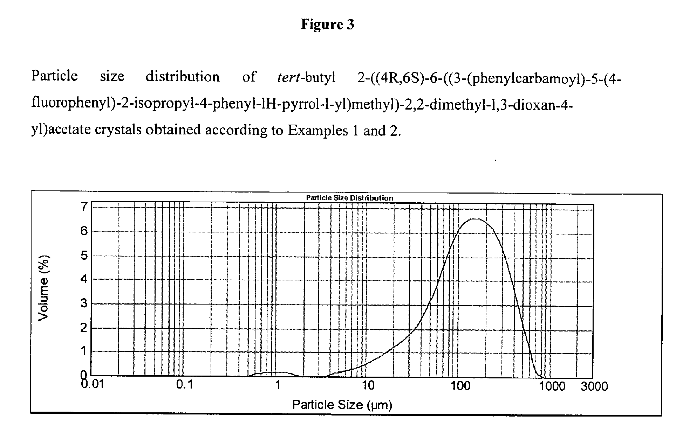

Microscopic pictures of the product are shown in Figure 1, and particle size distribution in Fig. 3.

### Example 3 - comparative example

The crystallization is carried out using 2-propanol as a medium in a reactor with mechanical stirring. 25g of crude *tert*-butyl 2-((4R,6S)-6-((3-(phenylcarbamoyl)-5-(4-fluorophenyl)-2-isopropyl-4-phenyl-1*H*-pyrrol-1-yl)methyl)-2,2-dimethyl-1,3-dioxan-4-yl)acetate is dissolved in 90 ml of 2-propanol at the reflux temperature of the mixture and the temperature is maintained for 30 minutes to allow all solid to dissolve. Then a linear cooling ramp -0.40 K/min of reactor jacket is applied and the mixture is cooled to 3 °C. The formed suspension is homogenized for 2 hours and the product is isolated using Büchner funnel in a suction filtration.

The time needed for the filtration of 10 mL of suspension on a surface of 0.79 cm² was approx. 120 s, at a vacuum of about 80-100 mbar.

This example was performed in the same equipment and using the same parameters/ as Example 1, only without the addition of CTABr.

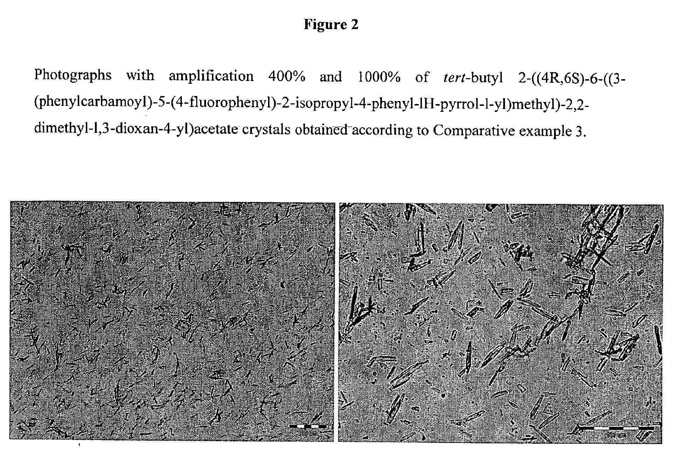

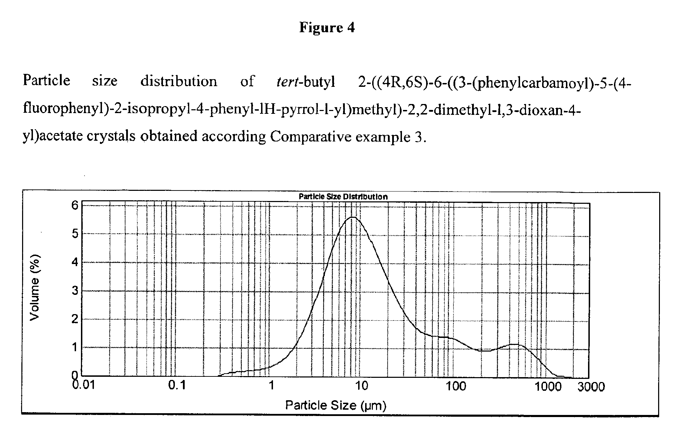

Microscopic pictures of the product are shown in Figure 2, and particle size distribution in Fig. 4.

### II.) Compound Ie

### Example 4

The crystallization is carried out using ethylacetate and water as crystallization media in a reactor with mechanical stirring. 5 g of crude lovastatin, (1S,3R,7S,8S,8aR)-8-[2-[(2R,4R)-4-hydroxy-6-oxotetrahydro-2*H*-pyran-2-yl]ethyl]-3,7-dimethyl-1,2,3,7,8,8a hexahydronaphthalen-1-yl (2S)-2-methylbutanoate), is dissolved in 25 ml of ethylacetate at the reflux. The solution is cooled to 65 °C and 1g of active carbon is added. The mixture isstirred at this temperature for the next 10 minutes. Then the active carbon is removed using filtration at the temperature of solution approximately 65 °C. To the above prepared, hot solution 20 ml of distilled water (with the temperature approximately 70 °C) and 1 g of cetyltrimethylammonium bromide is slowly added. Then the crystallization mixture is cooled to 50 °C. At this temperature the seeds of pure lovastatin (50 mg) are added and the temperature is maintained for the next 30 minutes. Finally, the formed suspension is slowly cooled to the 10 °C in the next 6 hours (dT/dt = -0.11 K/min). When the final temperature is reached the suspension is maintained at the temperature 10 °C for the next two hours. The formed product is isolated using vacuum filtration and washed with 15 ml of distilled water with room temperature.

### Example 5 - comparative example

The crystallization is carried out using ethylacetate and water as crystallization media in a reactor with mechanical stirring. 5 g of crude lovastatin, (1S,3R,7S,8S,8aR)-8-[2-[(2R,4R)-4-hydroxy-6-oxotetrahydro-2*H*-pyran-2-yl]ethyl]-3,7-dimethyl-1,2,3,7,8,8 a hexahydronaphthalen-1-yl (2S)-2-methylbutanoate), is dissolved in 25 ml of ethylacetate at the reflux. The solution is cooled to 65 °C and 1g of active carbon is added. The mixture is stirred at this temperature for the next 10 minutes. Then the active carbon is removed using filtration at the temperature of solution approximately 65 °C. To the above prepared, hot solution 20 ml of distilled water (with the temperature approximately 70 °C) is slowly added. Then the crystallization mixture was cooled to 50 °C. At this temperature the seeds of pure lovastatin (50 mg) are added and the temperature is maintained for the next 30 minutes. Finally, the formed suspension is slowly cooled to the 10 °C in the next 6 hours (dT/dt = -0.11 K/min). When the final temperature is reached the suspension is maintained at the temperature 10 °C for the next two hours. The formed product is isolated using vacuum filtration and washed with 15 ml of distilled water with room temperature.

| Compound | Structure | Name |
|---|---|---|
| Compound Ie | 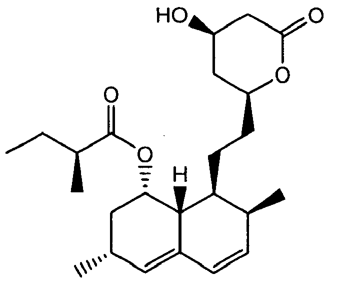 | (1*S*,3*R*,7S,8*S*,8a*R*)-8-[2-[(2*R*,4*R*)-4-Hydroxy-6-oxotetrahydro-2*H*-pyran-2-yl]ethyl]-3,7-dimethyl-1,2,3,7,8,8a-hexahydronaphthalen-1-yl (2*S*)-2-methylbutanoate |
| Imp. A | 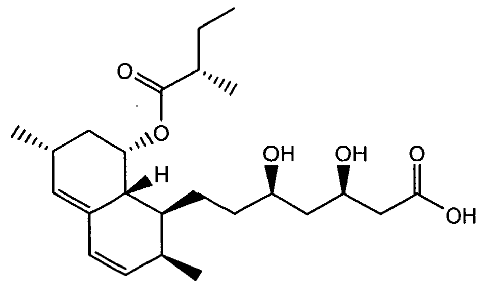 | (3R,5R)-7-[(1S,2S,6R,8S,8aR)-2,6-dimethyl-8-[[(2S)-2-methylbutanoyl]oxy]-1,2,6,7,8,8a-hexahydronaphthalen-1-yl]-3,5-dihydroxyheptanoic acid |
| Imp. B | 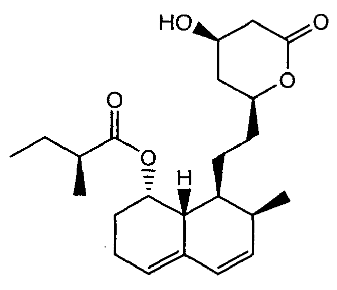 | (S)-((1S,7S,8S,8aR)-8-(2-((2S,4R)-4-hydroxy-6-oxotetrahydro-2H-pyran-2-yl)ethyl)-7-methyl-1,2,3,7,8,8a-hexahydronaphthalen-1-yl) 2-methylbutanoate |

| | Imp. A (w/w%) | Imp. B (w/w%) | Compound Ie (w/w %) |
|---|---|---|---|
| Starting material, crude compound Ie | 0.25 | 0.34 | 82 |
| Crystallized compound Ie , prepared according to Example 4 | 0.18 | n.d. | 98.9 |
| Crystallized compound Ie , prepared according to Example 5-comparative example | 0.30 | n.d. | 96.3 |

### III. Preparation of atorvastatin Ca

### Example 6

### Preparation of [R-(R*,R*)]-2-(4-fluorophenyl)-β,δ-dihvdroxy-5-(1-methylethyl) phenyl-4-[(phenylamino)carbonyl]-1H-pyrrole-1-heptanoic acid tertiary butyl ester

26,67 g of (4R-cis)-6-{2-[3-phenyl-4-(phenylcarbamoyl)-2-(4-fluorophenyl)-5-(1-methylethyl)pyrrol-1-yl]ethyl}-2,2-dimethyl-[1,3]dioxane-4-yl acetic acid tertiary butyl ester (Ia) is suspended in 120 mL of acetonitrile, 23 mL of water and 6.3 mL of 1M HCl. The reaction mixture is stirred at 20-25 °C until all (4R-cis)-6-{2-[3-phenyl-4-(phenylcarbamoyl)-2-(4-fluorophenyl)-5-(1-methylethyl)pyrrol-1-yl]ethyl}-2,2-dimethyl-[1,3]dioxane-4-yl acetic acid tertiary butyl ester (Ia) is dissolved and the reaction is followed with HPLC method. After 24 h 267 mL of water was added. The solid precipitate is filtered off and the cake is washed with 100 mL of water unitll the pH value of the filtrate is 5-7. The wet cake is dried at 20-30 °C on air for approximately 1 hour till constant weight, optionally granulated and dried for 4 hours at 40-50°C.
22,54 g of tertiary butyl ester [R-(R*,R*)]-2-(4-fluorophenyl)-β,δ-dihydroxy-5-(1-methylethyl)phenyl-4-[(phenylamino)carbonyl]-1*H*-pyrrole-1-heptanoic acid is isolated The purity of the obtained product is higher than 99.9%.

### Example 7

### Preparation of amorphous atorvastatin hemi-calcium

To the solution of 8,87 g [R-(R*,R*)]-2-(4-fluorophenyl)-β,δ-dihydroxy-5-(1-methylethyl)phenyl-4-[(phenylamino)carbonyl]-1*H*-pyrrole-1-heptanoic acid tertiary butyl ester in 31 mL of methanol and 26 mL *tert*-butyl methyl ether, 0.60 g of NaOH and 30 mL of water are added. The reaction mixture is purged with nitrogen flow for cca. 5 minutes, heated to the reflux for 2-4 h, until the concentration of the starting compound [R-(R*,R*)]-2-(4-fluorophenyl)-β,δ-dihydroxy-5-(1-methylethyl)phenyl-4-[(phenylamino)carbonyl]-1*H-*pyrrole-1-heptanoic acid tertiary butyl ester is lower than 0.5 %, determinated by HPLC.
The reaction mixture is allowed to cool to 20-25 °C, and filtered over randalite in methanol. 3% (w/w) of active charcoal suspended in water are added, the reaction mixture is heated to 50°C, cooled back to room temperature and filtered. The pH is set to 8.0-8.2 with the addition of aqueous HCl. The reaction mixture is washed with 3 × 60 mL *tert*-butyl methyl ether and aqueous parts are finally filtered. The reaction mixture is purged with nitrogen flow for cca. 5 min, and 1.66 g of CaCl₂ x6H₂O) and 31 mL of water is added in a 15-20 min interval at 20-25 °C. After the complete addition of CaCl₂ to the reaction mixture, the reaction mixture is stirred for additional 15-30 min; and 250 mL of water is slowly added into the reaction mixture to provoke the solidification of the thick emulsion-like mixture. Hemi-calcium salt of atorvastatin is formed and filtered off. The wet cake is washed with a mixture of water and methanol and finally with water. The collected solid material is dried on air to obtain dry solid atorvastatin hemi-calcium.

The dry hemi-calcium salt of atorvastatin can optionally be additionally milled on a dry pearl mill.

## Claims

1. A process for the crystallization of a compound of formula Ia-Ie: 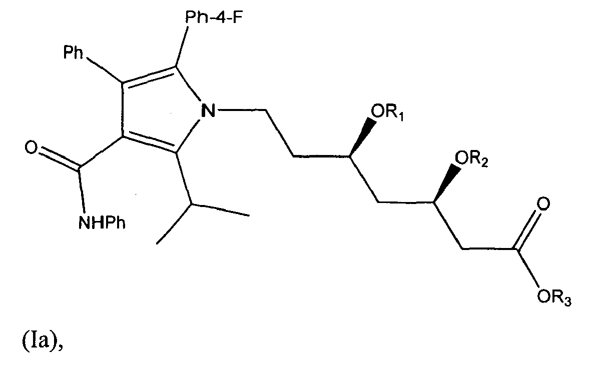 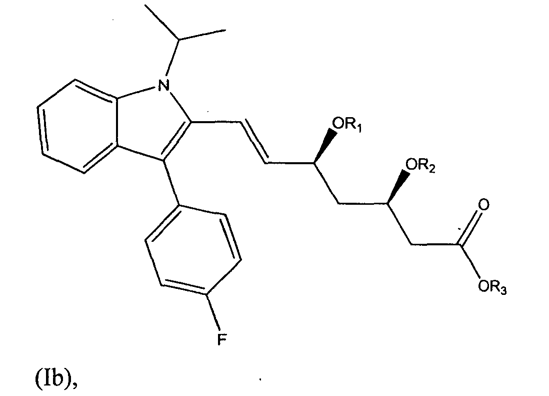 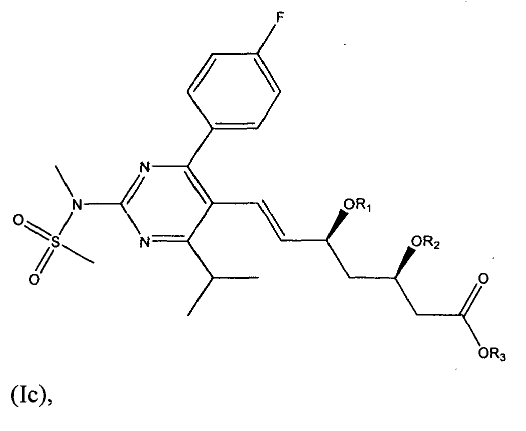 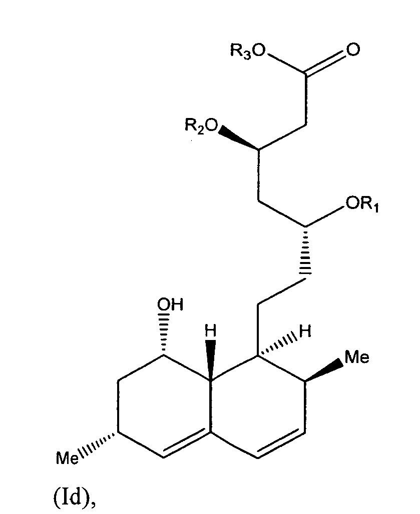 and 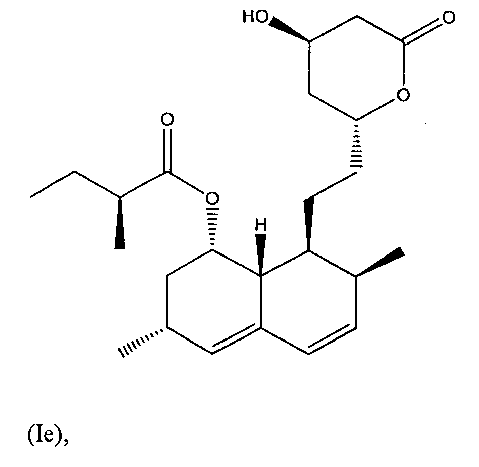 wherein R₁ and R₂ may be the same or different and are selected from any one or more of H, C₁-C₆ alkyl which may be straight or branched, unsubstituted or substituted with a halogen group, C₁-C₆ alkoxy group, or R₁ and R₂ together represent an alkylidene group of the formula CRₐR_{b} wherein Rₐ and R_{b} may be the same or different and are selected from any one or more of a C₁-C₁₁ alkyl group, and R₃ is selected from any one or more of a C₁-C₆ alkyl group; and in case of compound Id may also represent a hydrogen atom, in the presence of an amphiphilic additive.

2. A process according to claims 1, **characterized in that** the amine is a quarternary amine salt, preferably cetyltrimethylammonium bromide or cetyltrimethylammonium chloride.

3. A process according to claims 1 to 6, **characterized in that** the compound Ia is *tert*-butyl 2-((4R,6S)-6-((3-(phenylcarbaiuoyl)-5-(4-fluorophenyl)-2-isopropyl-4-phenyl-1*H*-pyrrol-1-yl)methyl)-2,2-dimethyl-1,3-dioxan-4-yl)acetate.

4. A process according to claim 7 **characterized in that** the solvent is selected from any one or more of toluene or other aromatic hydrocarbons, tetrahydrofurane, or cyclic and acyclic ethers, ethyl acetate and other esters of alcohols with carboxylic acids, 2- butanone, acetone, and other cyclic and acyclic ketones, methanol, etanol, *iso*-propanol, *n*-propanol, *iso*-butanol, *sec*-butanol, *n*-butanol and other alcohols, acetonitrile, DMSO, DMF and other amides, hexane, heptane, and other acyclic and cyclic aliphatic hydrocarbons, aliphatic nitriles, or binary mixtures thereof, preferably isopropanol or ethyl acetate.

5. A process according to claims 1 to 6, **characterized in that** the compound Ia is [R-(R*,R*)]-2-(4-fluorophenyl)-β,δ-dihydroxy-5-(1-methylethyl) phenyl-4-[(phenylamino)carbonyl]-1*H*-pyrrole-1-heptanoic acid tertiary butyl ester.

6. A process according to claims 1 to 6, **characterized in that** the compound Ic is *tert-*butyl 2-((4R,6S)-6-((E)-2-(4-(4-fluorophenyl)-6-isopropyl-2-(N-methylmethylsulfonamido)pyrimidin-5-yl)vinyl)-2,2-dimethyl-1,3-dioxan-4-yl)acetate.

7. A process according to claims 1 to 6, **characterized in that** the compound Id is (3R,5R)-3,5-dihydroxy-7-((1S,2S,6R,8S,8aR)-8-hydroxy-2,6-dimethyl-1,2,6,7,8,8a-hexahydronaphthalen-1-yl)heptanoic acid.

8. A process according to claims 1 to 6, **characterized in that** the compound Ie is lovastatin.

9. A process according to claims 1 to 14 **characterized in that** the percentage of the amphiphilic compound used with regard to compound Ia to Ie is between 0.01% (w/w) and 20% (w/w).

10. Use of cetyltrimethylammonium bromide for the purification of *tert*-butyl 2-((4R,6S)-6-((3-(phenylcarbamoyl)-5-(4-fluorophenyl)-2-isopropyl-4-phenyl-1*H*-pyrrol-1-yl)methyl)-2,2-dimethyl-1,3-dioxan-4-yl)acetate, [R-(R*,R*)]-2-(4-fluorophenyl)-β,δ-dihydroxy-5-(1-methylethyl) phenyl-4-[(phenylamino)carbonyl]-1*H*-pyrrole-1-heptanoic acid tertiary butyl ester, *tert-*butyl 2-((4R,6S)-6-((E)-2-(4-(4-fluorophenyl)-6-isopropyl-2-(N-methylmethylsulfonamido)pyrimidin-5-yl)vinyl)-2,2-dimethyl-1,3-dioxan-4-yl)acetate, (3R,5R)-3,5-dihydroxy-7-((1S,2S,6R,8S,8aR)-8-hydroxy-2,6-dimethyl-1,2,6,7,8,8a-hexahydronaphthalen-1-yl)heptanoic acid, lovastatin or 2-((4R,6S)-6-((E)-2-(3-(4-fluorophenyl)-1-isopropyl-1H-indol-2-yl)vinyl)-2,2-dimethyl-1,3-dioxan-4-yl)acetate.

11. *Tert*-butyl 2-((4R,6S)-6-((3-(phenylcarbamoyl)-5-(4-fluorophenyl)-2- isopropyl-4-phenyl-1*H*-pyrrol-1-yl)methyl)-2,2-dimethyl-1,3-dioxan-4-yl)acetate containing less than 0.10%(w/w) of Imp1.

12. *Tert*-butyl 2-((4R,6S)-6-((3-(phenylcarbamoyl)-5-(4-fluorophenyl)-2- isopropyl-4-phenyl-1*H*-pyrrol-1-yl)methyl)-2,2-dimethyl-1,3-dioxan-4-yl)acetate containing less than 0.10 % (w/w) of Imp2.

13. Use of amphiphilic compounds as promotors for crystal growth in the crystallization of statin intermediates

14. Use of amphiphilic compounds for crystallizations for production of materials with good filtrations properties.

15. Use of cetyltrimethylammonium bromide for the preparation of atorvastatin hemi-calcium.
